(19) Europäisches Patentamt
European Patent Office
Office européen des brevets

(11) Veröffentlichungsnummer: **0 173 950**
**A2**

(12) # EUROPÄISCHE PATENTANMELDUNG

(21) Anmeldenummer: 85110755.7

(22) Anmeldetag: 27.08.85

(51) Int. Cl.[4]: **C 07 H 15/22**
C 12 P 19/28, A 61 K 31/70
//(C12P19/28, C12R1:465)

(30) Priorität: 04.09.84 DE 3432432

(43) Veröffentlichungstag der Anmeldung:
12.03.86 Patentblatt 86/11

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Vértesy, László, Dr.
Eppenhainer Weg 6
D-6239 Eppstein/Taunus(DE)

(72) Erfinder: Bender, Rudolf, Dr.
Viktoriastrasse 6
D-6242 Kronberg/Taunus(DE)

(72) Erfinder: Fehlhaber, Hans-Wolfram, Dr.
Thomas-Mann-Strasse 5a
D-6270 Idstein/Taunus(DE)

(54) **Neuer alpha-Glukosidase Inhibitor, Verfahren zur seiner Herstellung, seine Verwendung und pharmazeutische Präparate.**

(57) Pseudooligosaccharid der Formel I

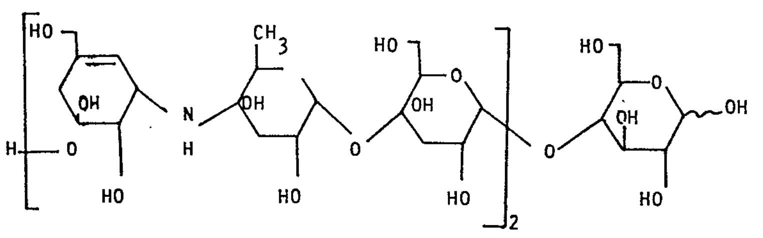

I

und dessen physiologisch verträgliche Salze mit Säuren, Verfahren zur Herstellung, pharmazeutische Präparate sowie die Verwendung werden beschrieben. Die Verbindung besitzt α-Glukosidase-hemmnede Wirkung.

EP 0 173 950 A2

Croydon Printing Company Ltd.

HOECHST AKTIENGESELLSCHAFT HOE 84/F 206 Dr.D/mü

Neuer α-Glukosidase Inhibitor, Verfahren zu seiner Herstellung, seine Verwendung und pharmazeutische Präparate

Die Erfindung betrifft ein neues, biologisch aktives Pseudooligosaccharid und dessen physiologisch verträgliche Salze. Es besitzt α-Glukosidase-, d.h. z.B. α-Amylase- und Disaccharidase-hemmende Eigenschaften und kann daher in der Human- und Tiermedizin, in der Tierernährung sowie in der Biotechnologie der Stärke verwendet werden.

Das erfindungsgemäße Pseudooligosaccharid weist folgende Formel I auf

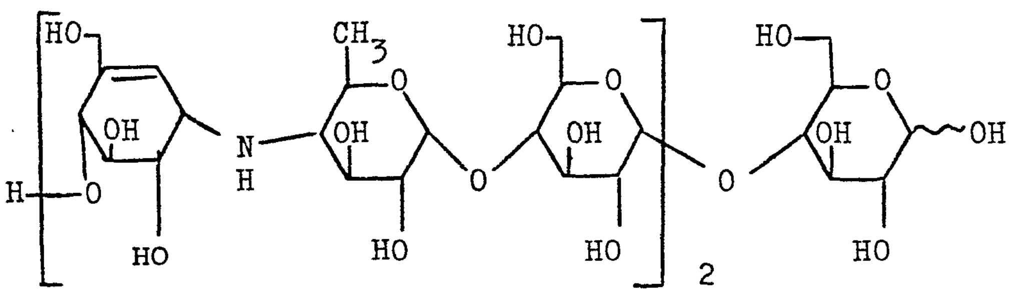

I

Es besitzt basischen Charakter sowie reduzierende Eigenschaften und entsprechend der Summenzusammensetzung $C_{44}H_{74}N_2O_{30}$ ein Molekulargewicht von 1110. Dieses Pseudooligosaccharid wird in den folgenden Ausführungen auch als Inhibitor AI-5662 bezeichnet. Die Erfindung betrifft den Inhibitor AI-5662 sowie dessen physiologisch verträgliche Salze mit Säuren.

Die Erfindung betrifft ferner ein Verfahren zur Herstellung des Pseudooligosaccharids der Formel I, pharmazeutische Präparate enthaltend eine Verbindung der Formel I, sowie die Verwendung als Arzneimittel, Diagnostikum und Reagenz.

Das Verfahren zur Herstellung des Pseudooligosaccharids der Formel I ist dadurch gekennzeichnet, daß man einen das Pseudooligosaccharid der Formel I erzeugenden Streptomyceten in einem Fermentationsmedium im submersverfahren kultiviert, den Inhibitor aus dem Mycel oder dem Kulturfiltrat

in an sich bekannter Weise isoliert und reinigt. Von den Streptomyceten ist Streptomyces nov. spec. FH 1717 geeignet. Dieser Stamm ist bei der Deutschen Sammlung von Mikroorganismen (DSM) unter der Registrier-Nr. DSM 3006 hinterlegt worden. Es können aber auch die Varianten und Mutanten dieses Stammes zur Gewinnung des Inhibitors AI-5662 eingesetzt werden.

Die taxonomischen Eigenschaften des Streptomyces FH 1717, DSM 3006 , können keiner Beschreibung eines Streptomyceten nach dem Bergly's Manual of Determinative Bacteriology, 8. Auflage, Verlag Williams & Wilkins Corp. Baltimore, 1974 oder anderer literaturbekannter Beschreibungen zugeordnet werden. Unterschiede zu beschriebenen Stämmen bestehen in wesentlichen physiologischen Merkmalen. Die folgende Tabelle 1 zeigt morphologische Kriterien und Ergebnisse von physiologischen Untersuchungen.

T a b e l l e 1

Identifizierung des Streptomyces novo species FH 1717

| | |
|---|---|
| Luftmycelfarbe | grau |
| Identifiziert als | Steptomyces species |
| Substratmycelfarbe | |
| Endopigment | schwarz |
| Exopigment | dunkel |
| Morphologie der Sporenkette | RA/S |
| Sporenoberfläche | glatt |
| Melaninbildung | |
| Komplex | + |
| Syntehtisch | + |
| C-Quellen-Verwertungs-Spektrum | |
| Glucose | + |
| Arabinose | + |
| Xylose | + |
| Fructose | (+) |
| Rhamnose | + |
| Lactose | - |
| Saccharose | (+) |

Tabelle 1 (Fortsetzung)

| | |
|---|---|
| Raffinose | (1) |
| Mannit | 1 |
| Inosit | - |
| **Säurereihe** | |
| Gluconat | + |
| Citrat | - |
| Malonat | - |
| Malat | + |
| Lactat | + |
| Oxalat | + |
| **Säurebildung** | - |
| **Verwertung von Makromolekülen** | |
| Gelatine | + |
| **Hämolyse** | -/- |
| **Eigelb-Reaktion** | |
| Lecithovitellin-reaktion | - |
| Pearly layer | 5 |
| Proteolyse | 7 |
| **Harnstoffspaltung** | + |
| **Nitrat** 1 g | + |
| **Nitrit** 1 g | + |
| " 3 g | - |
| **Lysozym** (ug/ml) | |
| 0 | + |
| 10 | - |
| 50 | - |
| 100 | - |
| **Allantoin-Abbau** | 1 |
| **Aesculin-Spaltung** | + |
| **Hippursäure-Spaltung** | (+) |
| **Voges-Proskauer-Reaktion** | 1 |
| **NaCl-Resistenz** (%) | |
| 4 | + |
| 7 | - |
| 10 | - |
| 13 | - |
| **Antibiotische Aktivität (Hemmung in mm)** | |
| Escherichia coli DSM 682 | - |
| Pseudomonas aeruginosa " 50 071 | - |
| Corynebacterium bovis | - |

Tabelle 1 (Fortsetzung)

| | | |
|---|---|---|
| Staphylococcus aureus | DSM 346 | - |
| Bacillus cereus | " 486 | - |
| Mucor ramannianis | NRRL 1839 | - |
| Candida albicans | " Y-477 | - |
| Temperatur-Bereich | | |
| 20 | | + |
| 28 | | + |
| 37 | | + |
| 45 | | 7 Kol |
| 55 | | - |

+ Wachstum oder pos. Reaktion, (+) schwaches Wachstum, I sehr schwaches Wachstum, - negative Reaktion.

Streptomyces species wie in der Tabelle 1 wiedergegeben, sind in der Literatur noch nicht beschrieben. Folglich ist der Steptomyces FH 1717 neu. Die Erfindung betrifft daher auch den Steptomyces novo species FH 1717, DSM 3006.

Bei der Gewinnung des Inhibitors AI-5662 geht man zweckmäßig wie folgt vor:

Streptomyces novo spec. FH 1717 wird in einem wäßrigen Nährmedium unter submersen und vorzugsweise aeroben Bedingungen gezüchtet, bis man eine ausreichende Konzentration des Inhibitors AI-5662 erhält. Das Nährmedium enthält einerseits Kohlenstoffquellen, wie beispielsweise Kohlenhydrate, andererseits Nährsalze und Stickstoffquellen, wozu geeignete Stickstoffverbindungen zählen, z.B. proteinhaltige Materialien. Bevorzugte Kohlenstoff liefernde Verbindungen sind Glucose, Saccharose, Glycerin, Malzextrakt, Stärke, Öle, Fette und dergleichen. Bevorzugte Stickstoff liefernde Substanzen sind beispielsweise Maisquellwasser, Hefeextrakte, Sojamehl, Fischmehl, Magermilchpulver, angedautes Casein oder Fleischextrakt. Es können auch sogenannte "synthetische" Nährlösungen verwendet werden. Es kann weiterhin nützlich sein, Spurenelemente, wie z.B. Zink, Magnesium, Eisen, Kobalt oder Mangan dem Fermentationsmedium zuzugeben.

Die Fermentation, die zur Bildung des Inhibitors AI-5662 führt, kann in einem weiten Temperaturbereich durchgeführt werden. Beispielsweise wird sie bei Temperaturen zwischen 10 und 40°C, vorzugsweise zwischen ungefähr 20 und 35°C durchgeführt. Den pH-Wert des Mediums hält man ebenfalls bei Werten, die dem Wachstum der Mikroorganismen föderlich sind, beispielsweise bei pH-Werten zwischen 4,0 und 10,0, vorzugsweise zwischen 6,0 und 9,5. In Abhängigkeit vom Nährmedium, wie z.B. seiner qualitativen und quantitativen Zusammensetzung und den Fermentationsbedingungen, wie z.B. Belüftungsrate, Temperatur oder pH-Wert, erfolgt die Bildung des AI-5662 in der Kulturlösung gewöhnlich nach etwa 1 - 10 Tagen.

Der Inhibitor AI-5662 befindet sich sowohl im Mycel als auch im Kulturfiltrat der Fermentation. Die Hauptmenge des AI-5662 ist im allgemeinen im Kulturfiltrat zu finden. Deshalb wird vorteilhafterweise die wäßrige Phase vom Mycel, beispielsweise durch Filtration oder Zentrifugation getrennt und das Pseudooligosaccharid aus den jeweiligen Phasen durch an sich bekannte Verfahren isoliert und gereinigt. Hierzu eignet sich eine große Zahl von Verfahren, wie beispielsweise Chromatographie an anorganischen Trägern wie $Al_2O_3$ oder Kieselgel sowie Trennungen an Ionenaustauschern, Molekularsieben oder Adsorptionsharzen, Lösungsmittel- bzw. Salzfällungen, Ultrafiltration, Craig-Verteilung u.a.

Ein bevorzugtes Verfahren zur Gewinnung des AI-5662 besteht darin, daß man den Inhibitor aus dme Kulturfiltrat an ein geeignetes Harz, z.B. auf Polystyrolbasis, adsorbiert, dieses beladene Harz abtrennt und den Inhibitor AI-5662 durch Elution mit geeigneten Pufferlösungen wie z.B. Phsophat- oder Na-acetat-Pufferlösung oder mit ggf. wasserhaltigen organischen Lösungsmitteln, wie z.B. Methanol, Ethanol, Aceton vorzugsweise aber mit wäßrigem Isopropanol isoliert. Die inhibitorhaltigen Eluate konzentriert man durch Ultrafiltration in bekannter Weise, wobei gleichzeitig eine Entsalzung vorgenommen wird. Die ionenarme wäßrige Lösung des AI-5662 wird dann auf einer Ionenaustauscher-Säule chromatographisch in an sich bekannter Weise aufgetrennt. Vorzugsweise verwendet man stark oder schwach saure Kationenaustauscher z.B. auf Styrol-Divinylbenzol-Copolmerisatbasis, welche als funktionelle Gruppen $-SO_3H$ oder $-COOH$ Gruppen tragen (®Dowex 50, ®Amberlite CG 120) oder auf Basis modifizierter Sulfopropyl-cellulose (SP-®Sephadex) als Ionenaustauscher, brauchbar sind aber auch eine große Anzahl anderer käuflicher Kationenaustausch. Der nächste Schritt der Reinigung ist die Anwendung eines Molekularsiebes, wie z.B. auf Polyacrylamid-Gel-Basis (®Biogel P-6) oder auf Basis modifizierter Cellulose (®Sephadex G-25). Durch Sammeln des Molekularbereichs um 1 100 Da erhält man den α-Amylasehemmstoff AI-5662. Es kann aber vorkommen, daß die, nach dem genannten Verfahren gewonnene Substanz noch geringe Verunreinigungen enthält. Es ist in solchen Fällen nützlich, als Nachreinigung eine weitere Trennung an Kieselgel durchzuführen. Als Elutionsmittel dient zweckmäßigerweise eine Mischung von n-Propanol-Ethylacetat-Wasser-Eisessig im Mengenverhältnis 6 : 1 : 3 : 0,5. Die inhibitorhaltigen Eluate werden vereinigt, im Vakuum eingeeengt und gefriergetrocknet. Die spezifische Aktivität eines solchen Produkts beträgt $4 \times 10^4$ α-Amylaseinhibitoreinheiten per mg.

Der reine Inhibitor AI-5662 ist ein farbloses, amorphes Pseudooligosaccharid. Es enthält Stickstoff und hat schwach basischen Charakter. So wandert der Inhibitor AI-5662 in der Hochspannungselektrophorese in sauren Puffern, wie z.B. wäßrigen Ameisensäure/Essigsäure-Mischungen als Kation in Richtung Kathode. Die erfindungsgemäße Substanz beeinhaltet Glukose in gebundener Form: durch ihre saure Hydrolyse entsteht Glucose neben anderen, zu meist stickstoffhaltigen Spalt-Produkten. Es ist weiter kennzeichnend für den Inhibitor AI-5662, daß er reduzierende Eigenschaft besitzt, die, wie in der Zuckerchemie üblich, z.B. mit

Triphenyltetrazoliumchlorid (TTC) nachgewiesen werden kann. Die erfindungsgemäße Verbindung hat die oben angegebene Formel. Die Substanz ist in Wasser leicht löslich. Das Absorptionsspektrum des ultravioletten Lichts, von 400 - 210 nm in wäßriger Lösung gemessen, zeigt eine Endabsorption bei der niedrigen Wellenlänge. Das Infrarotspektrum (aufgenommen in Br) ist in der Figur wiedergegeben.

In der Literatur sind bereits mehrere α-Glukosidaseinhibitoren mit Pseudooligosaccharidcharakter beschrieben worden E. Truscheit et al., Angew. Chem. 93, Seite 738 - 755 (1981), T. Tajiri et al., Agric. Biol. Chem. 47, Seite 671 - 679 (1983), K. Yokose et al., J. Antibiotics, 36, Seite 1157 - 1175 (1983).

Durch seine Strukturformel I aber auch z.T. durch die reduzierende Eigenschaft unterscheidet sich der erfindungsgemäße Inhibitor von allen bekannten α-Glukosidaseinhibitoren, somit liegt hier eine neue Substanz vor, die mikrobiologisch in guten Ausbeuten zu gewinnen ist.

Die α-Amylase- und Disaccharidase-hemmenden Eigenschaften des erfindungsgemäßen Inhibitors AI-5662 sind im Hinblick auf die Anwendung als Therapeutikum gegen Diabetes und Prädiabetes sowie Adipositas und Unterstützung von Diät interessant. Aufgrund seiner Eigenschaften ist er auch als Reagenz für diagnostische Zwecke wertvoll.

Stärkehaltige Nahrungs- und Genußmittel führen bei Tier und Mensch zu einem Anstieg des Blutzuckers und dadurch auch zu einer vermehrten Insulinsekretion des Pankreas. Die Hyperglykämie kommt durch die Aufspaltung der Stärke im Verdauungstrakt unter Einwirkung von Amylase und Maltase zu Glukose zustande.

Bei Diabetikern ist die Hyperglykämie besonders ausgeprägt und langanhaltend.

Die alimentäre Hyperglykämie sowie die Hyperinsulinämie nach Stärkeaufnahme läßt sich durch den erfindungsgemäßen α-Glukosidase-Inhibitor AI-5662 vermindern. Diese Wirkung ist dosisabhängig. Der erindungsgemäße Amylase-Inhibitor läßt sich daher als Therapeutikum einsetzten bei Diabetes, Prädiabetes und Adipositas sowie zur Unterstützung von Diät. Zu diesem Zweck empfiehlt sich eine orale Verabreichung, insbesondere zu den Mahlzeiten. Die Dosierung, die sich nach dem Gewicht des Patienten sowie dem individuellen Bedarf ausrichten soll, beträgt etwa 5 - 300 mg pro Dosis, die zweckmäßig zu jeder Mahlzeit genommen wird. Die Dosierung kann jedoch in begründeten Einzelfällen auch darüber oder darunter liegen.

Der erfindungsgemäße α-Glukosidase-Inhibitor eignet sich insbesondere zur oralen Verabreichung. Es kann als reine Substanz, als physiologisch verträgliches Salz mit Säuren, aber auch in Form einer pharmazeutischen Zubereitung unter Verwendung der üblichen Hilfs- und Trägerstoffe angewandt werden. Auch eine kombinierte Anwendung mit anderen Arzneimitteln wie blutzuckersenkenden oder lipidsenkenden Substanzen kann von Vorteil sein. Da höhermolekulare Saccharide als solche nicht oder nicht nennenswert aus dem Verdauungstrakt resorbiert werden, sind von der erfindungsgemäßen Substanz keine toxikologisch bedenklichen Nebenwirkungen zu erwarten. Dementsprechend konnten nach oraler Gabe auch hoher Dosen des α-Glukosidase-Inhibitors AI-5662 an Versuchstieren keine auffälligen Symptome erkannt werden. Zur Prüfung der pharmakologischen Wirkung des AI-5662 erhielten nüchterne, männliche Wistar-Ratte mit einem Gewicht zwischen 200 und 250 g den erfindungsgemäßen Hemmstoff AI-5662 gleichzeitig mit 2 g Stärke pro kg Körpergewicht oral verabreicht. Durch Bestimmung von Blutzuckerkonzentrationen in Blutproben, die vor, während und nach der Applikation des α-Amylaseinhibitors entnommen worden

sind, wurde die Wirksamkeit des Präparates bewiesen. Neben der Blutglukoseregulation kann man die erfindungsgemäßen Polypeptide auch zur Hemmung der Speichel-α-Amylase verwenden. Dieses Enzym bewirkt die Verdauung der Stärke im Mund und der so gebildete Zucker fördert den Kariesbefall der Zähne. Die erfindungsgemäßen Verbindungen können daher zur Verhütung oder Verminderung der Ausbildung von Karies angewandt werden.

Weiterhin können sie als biochemische Reagenzien sowie als diagnostische Mittel verwendet werden.

Amylasetest

Ein Amylase-Inhibitoreinheit (AIE) ist defineirt als die Inhibitormenge, die unter den Testbedingungen zwei Amylase-Einheiten (AE) zu 50 % zu hemmen vermag. Eine Amylase-Einheit ist nach internationaler Übereinkunft die Enzymmenge, die in einer Minute 1 μ äuqivalent glucosidischer Bindungen in der Stärke spaltet. Die μVal gespaltener glucosidischer Bindungen werden als μVal reduzierender Zucker photometrisch mit Dinitrosalizylsäure bestimmt. Die Angaben sind als μMole Maltose berechnet, die anhand einer Maltose-Eichgeraden ermittelt werden.

Die Tests werden folgendermaßen durchgeführt:

α-Amylase aus Schweinepankreas und die zu testenden Lösungen werden gemeinsam in 1,0 ml 20 mM Phosphatpuffer, pH 6,9, + 10 mM NaCl 10 - 20 min. bei 37°C vorinkubiert. Die enzymatische Reaktion wird durch Zugabe von 1,0 ml löslicher Stärke (0,25 % in dem angegebenen Puffer) nach Zulkowski gestartet. Nach genau 10 min. wird die Reaktion mit 2,0 ml Dinitrosalizylsäure-Farbreagenz (nach Boehringer Mannheim: Biochemica-Information II) abgestoppt und zur Farbenentwicklung 5 min. im siedenden Wasserbad

0173950

erhitzt. Nach dem Abkühlen wird die Extinktion bei 546 nm gegen den Reagentienleerwert gemessen. Die 50 %ige Hemmung wird im Vergleich zur ungehemmten Enzymreaktion durch Einsatz verschiedener Inhibitormengen graphisch mittels der Wahrscheinlichkeitsauftragung bestimmt.

Beispiel 1

Zur Gewinnung von AI-5662 erfolgte die Imfpstoffanzucht (Anzucht des Mikroorganismus) - wie in der mikrobiologischen Praxis üblich - aus einer gefriergetrockneten Dauerform des Organismus Streptomyces novo species FH 1717, DSM 3006, über Einzelkoloniepassage und Schrägröhrchen. Die für die Fermentation notwendige Massenproduktion an Sporen wurde ebenfalls auf festem Nährboden in Roux-Flaschen durchgeführt.

Agarmedium für Platte, Schrägröhrchen und Roux-Flasche:

40 g Haferflocken wurden fein gemahlen, mit 950 ml Leitungswasser versetzt und 5 Minuten mit einem Ultra-Turrax homogenisiert. Anschließend setzte man 18 g Agar zu und sterilisierte 20 Minuten bei 120°C.

Das beimpfte Röhrchen und die Roux-Flasche wurde 5 Tage bei 28°C bebrütet und danach bei +4°C gehalten. Die Sporen wurden mit 10 ml sterilisiertem Aqua dest. oder physiologischer Kochsalzlösung von dem festen Nährboden abgeschwemmt. 5 ml der Suspension dienten zur Beimpfung eines 2000 ml Erlenmeyerkolbens, der mit 500 ml sterilisierter wäßriger Nährlösung mit einem pH-Wert von 7,3 und folgender Zusammensetzung beschickt war (Angaben in Gewichts-%)

1,00 % Glukose
0,40 % Caseinpepton
0,40 % Fleischextrakt

| | |
|---|---|
| 0,25 % | NaCl |
| 0,05 % | Hefeextrakt |
| 0,05 % | Leberpulver. |

Der Kolben wurde bei 220 Upm 24 Stunden bei +28°C auf einer Schüttelmaschine geschüttelt. Danach ist diese Vorkultur in einen 12 l-Braun-Fermenter überführt worden, der mit 9 l sterilisierter wäßriger Nährlösung beschickt war und einen pH-Wert von 7,4 hatte. Die Zusammensetzung der Nährlösung für die Hauptkultur war folgende (Angaben in Gewichts-%).

| | |
|---|---|
| 2,0 % | Fleischextrakt |
| 2,0 % | Malzextrakt |
| 1,0 % | Calciumcarbonat |
| 0,1 % | Antischaummittel |
| ad 100 % | Wasser. |

Die Hauptkultur wurde bie 28°C, 4 Tage mit 500 Upm gerührt die Luftzufur betrug 120 l pro Stunde. Nach 24, 48, 60, 72, 84 und 96 Stunden wurde der Gehalt an α-Glukosidase-Inhibitor gemäß der Vorschrift von R. Bender et al., Anal. Biochem., 137, 307 - 312 (1984) bestimmt. Der Stamm Str. nov. spec. FH 1717 lieferte unter den beschriebenen Versuchs- und Kulturbedingungen durchschnittlich $3 \times 10^3$ AEI/ml bei einem End-pH von 8,8.

Beispiel 2

8 l Fermentationslösung gemäß Beispiel 1 wurden mit Hilfe einer Zentrifuge von der Zellmasse befreit und die klare flüssige Phase wurde auf pH 9,5 eingestellt. Anschließend trug man die Lösung auf eine 0,8 l Polystyrol-Adsorptionsharz (Diaion® HP-20) beinhaltete Säule auf, wusch mit 1,5 l Wasser nach und eluierte mit Wasser, dem steigende Mengen Isopropanol hinzugefügt worden war. Die Mischung, die 5 % Isopropanol enthielt, löste den Inhibitor AI-5662

von der Säule. Diese aktiven Eluate (1,5 l) wurden durch Ultrafiltration konzentriert und unte Wasserzugabe sowie weitere Ultrafiltration solange entsalzt, bis das Retenat keine nachweislichen Salze mehr enthielt. Das resultierende Konzentrat (0,2 l) trennte man auf Sulfopropyl modif. Cellulose (SP-Sephadex ® ), die in die Säureform ($H^+$-Form) überführt war. Durch Anlegen eines Ammonium-acetatgradienten, pH 5 (0 - 1 molar), wurden die die α-Amylase-hemmende Aktivität enthaltenden Fraktionen eluiert. Die entsprechenden Fraktionen wurden in Ultrafiltrationszellen ( ®Amicon) konzentriert und entsalzt. Die gefriergetrocknete, aktive Substanz wurde hiernach auf eine, mit Kieselgel gefüllte Säule (3 x 35 cm) aufgetragen und mit einer Mischung von n-Propanol-Ethylacetat-Wasser-Eisessig (6 : 1 : 3 : 0,5) unter Druckanwendung eluiert. Das aktive Eluat wurde im Vakuum vom Lösungsmittel befreit und anschließend, in Wasser aufgenommen, auf pH 9 gestellt und auf Biogel P-6 der Endreinigung unterworfen. Das Säulenfließmittel war hierbei reines Wasser. Das gewonnene, aktive Material wurde gefriergetrocknet: Es resultieren 0,8 g eines weißen, amorphen Pulvers mit einer α-Amylase-hemmenden Wirkung von $4 \times 10^4$ AIE pro mg. In der Figur ist das IR-Spektrum in KBr dieses AI-5662 wiedergegeben. Die Elementaranalyse ergab 47,6 % Kohlenstoff, 6,7 % Wasserstoff, 2,5 % Stickstoff und 43,2 % Sauerstoff.

Beispiel 3

200 mg, des nach Beispiel 2 gewonnenen Inhibitors wurden in 1 ml Wasser gelöst und im Eisbad mit Schwefelsäure auf pH 2 gestellt. Anschließend wurde die Lösung mit Aceton auf 10 ml aufgefüllt, und 30 Minuten stehen gelassen, den entstandenen Niederschlage sammelte man durch Filtrieren. Der in Wasser gelöste und hiernach gefriergetrocknete Niederschlag erhab 204 mg des AI-5662-Sulfats.

Patentansprüche

1. Pseudooligosaccharid der Formel I

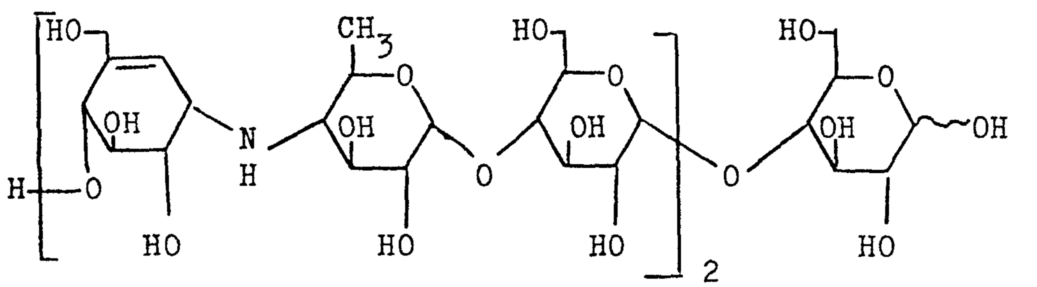

I

und dessen physiologisch verträgliche Salze mit Säuren.

2. Verfahren zur Herstellung des Pseudooligosaccharids gemäß Anspruch 1 und der physiologisch verträglichen Salze, dadurch gekennzeichnet, daß man einen das Pseudooligosaccharid der Formel I erzeugenden Streptomyceten in einem Fermentationsmedium im Submersverfahren kultiviert, das Pseudooligosaccharid aus dem Mycel oder Kulturfiltrat in an sich bekannter Weise isoliert und reinigt und gegebenenfalls mit einer Säure in ein physiologisch verträgliches Salz überführt.

3. Verfahren gemäß Anspruch 2, dadurch gekennzeichnet, daß man Streptomyces nov. sepc. FH 1717 kultiviert und aus dem Kulturfiltrat das Pseudooligosaccharid isoliert und reinigt.

4. Pharmazeutisches Präparat enthaltend das Pseudooligosaccharid gemäß Anspruch 1 oder ein physiologisch verträgliches Salz davon.

5. Verwendung des Pseudooligosaccharids gemäß Anspruch 1 oder ein physiologisch verträgliches Salz davon zur Hemmung der α-Glukosidase.

6. Verwendung einer Verbindung gemäß Anspruch 1 zur Behandlung von Diabetes, Prädiabetes und Adipositas.

7. Verwendung von Verbindungen gemäß Anspruch 1 als Diagnostikum, Reagenz oder Prophylaktikum gegen Karies.

8. Streptomyces nov. spec. FH 1717, DSM 3006.

Patentansprüche für Österreich:

1. Verfahren zur Herstellung des Pseudooligosaccharids der Formel I

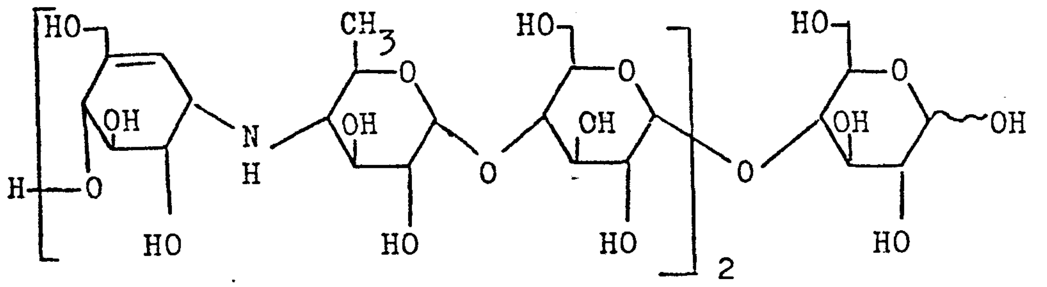

I

und von dessen physiologisch verträglichen Salzen mit Säuren, dadurch gekennzeichnet, daß man einen das Pseudooligosaccharid der Formel I erzeugenden Streptomyceten in einem Fermentationsmedium im Submersverfahren kultiviert, das Pseudooligosaccharid aus dem Mycel oder Kulturfiltrat in an sich bekannter Weise isoliert und reinigt und gegebenenfalls mit einer Säure in ein physiologisch verträgliches Salz überführt.

2. Verfahren gemäß Anspruch 1, dadurch gekennzeichnet, daß man Streptomyces nov. sepc. FH 1717 kultiviert und aus dem Kulturfiltrat das Pseudooligosaccharid isoliert und reinigt.

3. Verfahren zur Herstellung eines pharmazeutischen Präparats enthaltend das Pseudooligosaccharid der Formel I gemäß Anspruch 1 oder ein physiologisch verträgliches Salz davon, dadurch gekennzeichnet, daß man den Wirkstoff in eine geeignete Applikationsform überführt.

4. Verwendung des Pseudooligosaccharids der Formel I gemäß Anspruch 1 oder ein physiologisch verträgliches Salz davon zur Hemmung der α-Glukosidase.

5. Verwendung des Pseudooligosaccharids der Formel I gemäß Anspruch 1 oder ein physiologisch verträgliches Salz davon als Diagnostikum, Reagenz oder Prophylaktikum gegen Karies.

6. Streptomyces nov. spec. FH 1717, DSM 3006.

0173950

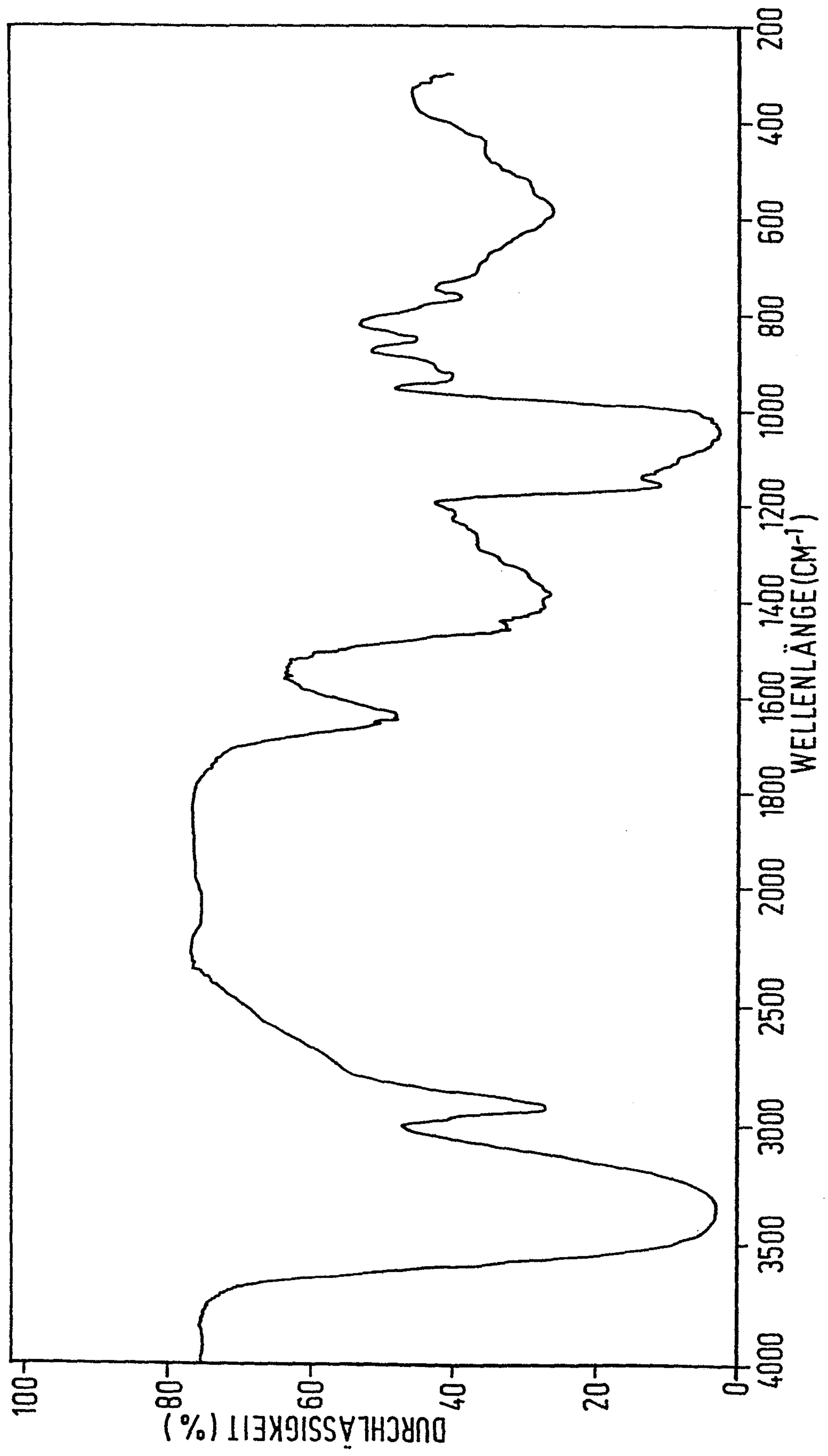

DURCHLÄSSIGKEIT (%)
100
80
60
40
20
0
4000
3500
3000
2500
2000
1800
1600
1400
1200
1000
800
600
400
200
WELLENLÄNGE (CM-1)